# EUROPÄISCHE PATENTANMELDUNG

(11) **EP 4 629 258 A1**
(43) Veröffentlichungstag der Anmeldung: **08.10.2025**
(21) Anmeldenummer: 24168053.7
(22) Anmeldetag: 02.04.2024
(51) Int. Cl.: G16H 20/17, G16H 40/20, G06F 3/023, G16H 40/67, H04L 67/12, H04L 67/55

(54) **BEHANDLUNGSZENTRUM ZUR DURCHFÜHRUNG VON MEDIZINISCHEN INFUSIONEN UND ZUGEHÖRIGES VERFAHREN**

(71) Anmelder: B. Braun Melsungen AG, 34212 Melsungen (DE)
(72) Erfinder: Kever, Felix, 34125 Kassel (DE)
(74) Vertreter: Tergau & Walkenhorst Intellectual Property GmbH

(57) **Zusammenfassung**

Behandlungszentrum (2) zur Durchführung von medizinischen Infusionen, umfassend:
a. ein Verwaltungssystem (6) mit einer Datenbank zur Speicherung von Patientendaten (12), wobei jedem Patienten (4) eine Patienten-ID (10) oder eine Fallnummer (8) zugeordnet ist,
b. ein Infusionspumpensystem (20) mit einem Backend (18), das über Datenverbindungen (16, 22) mit dem Verwaltungssystem (6) und mit einer Mehrzahl von Infusionspumpen (24) verbunden ist,
c. eine Mehrzahl von Infusionspumpen (24), jeweils mit einer Benutzeroberfläche einschließlich eines Eingabeelements (26), das die Eingabe eines Kurzkennzeichnungscodes (14), SVC genannt, ermöglicht.
d. eine Mehrzahl von körpergetragenen Identifikationsmerkmalen, insbesondere Patientenarmbändern (30), jeweils mit einem Patienten-SVC (14) beschriftet, zur temporären, eindeutigen Kennzeichnung von Patienten (4) für einen vorgegebenen Behandlungszeitraum, oder
eine Mehrzahl von jeweils einer der Infusionspumpen (24) zugeordneten Bettplätzen (32), jeweils mit einem eindeutigen Bettplatz-SVC (36) beschriftet,
wobei das Verwaltungssystem (6) und/oder das Backend (18) dazu ausgebildet ist, anhand eines an einer der Infusionspumpen (24) eingegebenen Patienten-SVCs (14) oder Bettplatz-SVCs (36) eine Zuordnung zwischen Patient (4) und Infusionspumpe (24) oder zwischen Bettplatz (32) und Infusionspumpe (24) vorzunehmen.

## Beschreibung

Die Erfindung betrifft ein Behandlungszentrum zur Durchführung von medizinischen Infusionen. Sie betrifft ferner ein Verfahren zum Betreiben eines Infusionspumpensystems.

Die sichere Zuordnung von medizinischen Geräten zum jeweiligen Patienten ist zwingend erforderlich, wenn es im Systemkontext zeitgleich mehr als einen Patienten gibt und die vom jeweiligen Gerät generierten Therapie-, Nutzungs- und Betriebsdaten in weiteren Systemen verarbeitet werden sollen. Anonyme Gerätedaten müssen ansonsten aufwändig rückwirkend dem richtigen Patienten zugeordnet oder verworfen werden.

Ein gängiges Verfahren für Infusionspumpen ist die indirekte Patientenzuordnung über den Bettplatz: Das übergeordnete Krankenhaus-Informations-System (KIS) oder Electronic Medical Record (EMR) System pflegt eine Liste von Bettplätzen, denen im Rahmen einer relationalen Datenbank temporär Patienten aus der Patientenliste zugeordnet werden. Wenn die Infusionspumpen nun ebenfalls einem Bettplatz zugeordnet werden und beide Zuordnungslisten laufend aktuell gehalten werden, ist eine eindeutige Zuordnung einer Pumpe zu einem Patienten jederzeit möglich.

Sofern die Zuordnung von Pumpe zu Bettplatz durch Übertragung der Bettplatz-Information von ortsfesten Racksystemen mit fester Bettplatz-Zuordnung erfolgt, funktioniert diese Methode zuverlässig und weitgehend automatisch. Schwieriger ist es mit Pumpen, die nicht mit Racksystemen, sondern als "mobile" Geräte genutzt werden: Hier gibt es in der Regel keine technische Lösung für eine automatische Zuordnung des aktuellen Bettplatzes, zudem unterliegen die Geräte angesichts ihrer geringen Größe und vergleichsweise großen Anzahl meist einer recht großen räumlichen Fluktuation.

Problematisch für die indirekte Patientenzuordnung können aber auch die Behandlungs-Workflows im Krankenhaus sein: In OPs oder auf Intensivstationen werden ganze Racksysteme spontan hinzugezogen (sind also nicht ortsfest und müssen zunächst dem neuen Bettplatz zugeordnet werden), während in Notsituationen Patienten infundiert werden müssen, die (noch) gar keinem Bettplatz zugeordnet sind - schlimmstenfalls auf dem Gang.

Aktuell bleibt in diesem Fall nur die Möglichkeit, die Pumpe vor jedem Infusionsstart manuell dem richtigen Bettplatz zuzuordnen - sei es über das lokale User Interface, das User Interface des Backends, oder ein SmartDevice mit Scanfunktion, welches mit dem Backend verbunden ist und als dessen Fernbedienung dient.

Ein mögliches Verfahren basiert auf der Patientenzuordnung über einen Auswahlprozess über das User Interface der Pumpe (bzw. allgemein: des Medizingerätes). Hierzu wird zunächst die Patientenliste (gegebenenfalls nur ein Auszug, gefiltert durch die am Gerät konfigurierte Care Unit) vom KIS heruntergeladen. Mit einer geeigneten Filterung, z. B. nach den Anfangsbuchstaben des Namens oder weiteren Kriterien, kann die Liste so verkürzt werden, dass sie eine praktikable / nutzerfreundliche Auswahl des Patienten ermöglicht. In jedem Fall erfordert die für eine erfolgreiche Zuordnung benötigte Datenbankabfrage / Filterung / Auswahl einige Nutzer-Interaktionen. Hinzu kommen datenschutzrechtliche Herausforderungen durch die Verarbeitung einer größeren Anzahl sensibler persönlicher Datensätze auf dem Medizingerät bzw. - bei umgekehrter Betrachtung - durch den über jede einzelne Infusionspumpe gegebenen Lesezugriff auf die gesamte Patientendatenbank.

In jedem Fall ist die Zuordnung mit einem gewissen Konfigurationsaufwand verbunden, der aufgrund der Relevanz der korrekten Zuordnung in Kombination mit der räumlichen Beweglichkeit der Infusionspumpen zwingend vom medizinischen Personal am Patientenbett geleistet werden muss und angesichts der üblicherweise gegebenen Arbeitsbelastung meist sehr kritisch gesehen wird.

Eine Aufgabe der Erfindung ist es, ein Behandlungszentrum zur Durchführung von medizinischen Infusionen und ein zugehöriges Verfahren zur einfachen, zuverlässigen und scannerlosen Patientenzuordnung von Infusionspumpen oder anderen medizinischen Geräten zu einem Patienten und/oder Bettplatz anzugeben, welches einfach zu implementieren ist und im klinischen Alltag eine hohe Flexibilität erlaubt.

Die genannte Aufgabe wird erfindungsgemäß gelöst durch ein Behandlungszentrum mit den Merkmalen von Anspruch 1 sowie durch ein Verfahren gemäß Anspruch 9.

Demnach ist ein Behandlungszentrum zur Durchführung von medizinischen Infusionen vorgesehen, umfassend:
a. ein Verwaltungssystem mit einer Datenbank zur Speicherung von Patientendaten, wobei jedem Patienten eine Patienten-ID oder eine Fallnummer zugeordnet ist,
b. ein Infusionspumpensystem mit einem Backend, das über Datenverbindungen mit dem Verwaltungssystem und mit einer Mehrzahl von Infusionspumpen verbunden ist,
c. eine Mehrzahl von Infusionspumpen, jeweils mit einer Benutzeroberfläche einschließlich eines Eingabeelements, das die Eingabe eines Kurzkennzeichnungscodes, SVC genannt, ermöglicht.
d. eine Mehrzahl von körpergetragenen Identifikationsmerkmalen, insbesondere Patientenarmbändern, jeweils mit einem Patienten-SVC beschriftet, zur temporären, eindeutigen Kennzeichnung von Patienten für einen vorgegebenen Behandlungszeitraum, oder
   eine Mehrzahl von jeweils einer der Infusionspumpen zugeordneten Bettplätzen, jeweils mit einem eindeutigen Bettplatz-SVC beschriftet,
   wobei das Verwaltungssystem und/oder das Backend dazu ausgebildet ist, anhand eines an einer der Infusionspumpen eingegebenen Patienten-SVCs oder Bettplatz-SVCs eine Zuordnung zwischen Patient und Infusionspumpe oder zwischen Bettplatz und Infusionspumpe vorzunehmen.

Die in Bezug auf die Vorrichtung genannten Aufgaben, Merkmale und Vorteile übertragen sich sinngemäß auf das Verfahren und umgekehrt.

Das hier beschriebene Konzept der Code-basierten Patientenzuordnung oder auf Englisch **Code-based Patient Assignment (CPA)** löst das oben beschriebene Problem mit Hilfe eines alphanumerischen Short Verification Code (SVC), welcher vom menschlichen Nutzer einfach und ohne technische Hilfsmittel zu erfassen und idealerweise über ein dediziertes Eingabeelement an der Infusionspumpe einzugeben ist. Das Eingabeelement kann insbesondere ein Soft-Keyboard sein, also eine Bildschirmtastatur auf einem Touchscreen der Infusionspumpe, welche bevorzugt speziell für den verwendeten SVC optimiert ist. Bereits ein dreistelliger alphanumerischer Code bietet rund 42.000 Varianten, was für die Abbildung der in einem großen Krankenhaus über die Zeit behandelten Patienten bzw. der diesen Patienten zugeordneten Fallnummern allerdings noch immer viel zu wenig ist.

Aus diesem Grund sieht das CPA-Konzept vor, dass der auf dem Patientenarmband aufgedruckte SVC über ein definiertes Zeitfenster mit der im KIS bzw. EMR-System (allgemein: im Verwaltungssystem) geführten, dauerhaft eindeutigen Fallnummer und Patienten-ID verknüpft ist. Es basiert also auf einer temporären und historisierten Zuordnung von SVC und Patienten-ID.

Beispielsweise ist der SCV "B3X" für einen Zeitraum von vier Wochen mit der Patienten-ID "08154711-1234" verknüpft - beginnend mit der Aufnahme im Krankenhaus. Um eine Infusionspumpe diesem Patienten zuzuordnen, muss nun lediglich dieser dreistellige SCV an der Pumpe eingegeben werden, was bei Vorhandensein eines Touchscreens mit Hilfe eines dedizierten Soft-Keyboards ebenso einfach wie nutzerfreundlich möglich ist. Die Pumpe sendet den SVC nun über das Backend des Infusionspumpen-Systems an das KIS oder EMR-System und erhält im Gegenzug die aktuell damit verknüpfte Patienten-ID sowie - optional - den Namen, das Alter sowie das Geschlecht des Patienten.

Die letztgenannten Metadaten werden - neben der ermittelten Patienten-ID - auf dem UI der Pumpe lediglich angezeigt, um dem Benutzer auf einfache Art ein zusätzliches Feedback zur Korrektheit der Zuordnung zu ermöglichen. Unmittelbar nach der positiven oder negativen Bestätigung des Benutzers werden diese Daten jedoch gelöscht; dauerhaft in der Pumpe gespeichert wird lediglich die Patienten-ID, welche nach dem Start der Infusion dann auch in dem an das EMR-System gesendeten Datenstrom enthalten ist.

Auch das Konzept der Code-basierten Standortzuordnung oder auf Englisch **Code-based Location Assignment (CLA)**, welches als Abwandlung oder Variante des CPA-Konzepts verstanden werden kann, löst das eingangs beschriebene Problem mit Hilfe eines alphanumerischen Short Verification Code (SVC), welcher vom menschlichen Nutzer einfach und ohne technische Hilfsmittel zu erfassen und idealerweise über ein dediziertes Soft-Keyboard an der Infusionspumpe einzugeben ist. Bereits ein zweistelliger alphanumerischer Code bietet rund 1.200 Varianten, was für die Abbildung der in einem Krankenhaus typischerweise vorhandenen Bettplätze ausreicht. Die rund 42.000 Varianten eines dreistelligen alphanumerischer Codes würden selbst das geschätzt größte Krankenhaus der Welt (Texas Medical Center, Houston) mit rund 9.200 Betten problemlos abdecken.

Für die Nutzung dieses Codes gibt es theoretisch drei Varianten, von denen zwei bevorzugt nutzbar sind - abhängig von der Architektur der Bettplatzzuordnung:

### Fall A:

Die Eingabe des Location-SVC erfolgt in/an der Pumpe; im Infusionspumpen-Backend wird die gemäß Hospital Structure zugehörige Bettplatz-Bezeichnung herausgesucht und im Erfolgsfall auf dem Pumpen-Display zur Bestätigung angezeigt (inkl. Metadaten). Nach erfolgter Bestätigung wird im Backend die Zuordnung zwischen Pumpe und Bettplatz vorgenommen und die Bettplatz-Bezeichnung zusammen mit den Infusionsdaten an die nachgelagerten Systeme übermittelt.

### Fall B:

Die Eingabe des Location-SVC erfolgt in/an der Pumpe; das Infusionspumpen-Backend fragt die dazugehörige Bettplatz-Bezeichnung beim übergeordneten EMR-System oder KIS an. Im Erfolgsfall wird diese auf dem Pumpen-Display zur Bestätigung angezeigt. Nach erfolgter Bestätigung wird die empfangene Bettplatz-Bezeichnung zusammen mit den Infusionsdaten an die nachgelagerten Systeme übermittelt.

### Fall C:

Die Eingabe des Location-SVC erfolgt in/an der Pumpe; dieser wird 1:1 zusammen mit den Infusionsdaten an die nachgelagerten Systeme übermittelt. Problem: Der User erhält keine Rückmeldung, auf deren Basis er die Korrektheit seiner Eingabe überprüfen oder wenigstens einschätzen könnte. Insofern ist diese Art der Bettplatzzuordnung relativ unsicher. Deswegen werden hier im Folgenden bevorzugt die Fälle A und B betrachtet.

Ergänzend wird Folgendes angemerkt: Welche Pumpen sich an welchen Bettplätzen befinden, "weiß" in der Regel nur das Infusionspumpensystem (also das Backend und/oder die einzelne Pumpe).

Die an das Verwaltungssystem geschickten Daten der Pumpen enthalten als Zusatzinformation den Bettplatz, damit das Verwaltungssystem sie dem richtigen Patienten zuordnen kann.

Zusätzlich enthalten sie die ID der Pumpe, so dass das Verwaltungssystem theoretisch auch weiß, welche Pumpen sich aktuell an welchem Bettplatz befinden. Dies ist aber keine notwendige Voraussetzung für die im Rahmen der vorliegenden Erfindung eigentlich adressierte Herausforderung (die Pumpendaten dem richtigen Patienten zuordnen).

Insofern ist der Regelfall, dass das Backend die Zuordnung zwischen Bettplätzen und Infusionspumpen gemäß den Bettplatz-SVCs verwaltet und aktualisiert.

### Allgemeine CPA- oder CLA-Voraussetzungen

Die hier beschriebenen Konzepte beruhen auf einer bestehenden Netzwerk- oder Datenverbindung zwischen Infusionspumpe, dem Backend des Infusionspumpen-Systems sowie dem KIS oder EMR-System (allgemein: dem Verwaltungssystem).

### CPA- oder CLA-bedingte Neuerungen

Grundlegend neu ist ein historisierend-patientenspezifischer oder bettplatzspezifischer, vorzugsweise alphanumerischer Short Verification Code (SVC), welcher einfach vom Patientenarmband oder von der Bettplatzbeschriftung abgelesen und über das User Interface der Infusionspumpe eingegeben werden kann. Mit lediglich drei alphanumerischen Zeichen (lateinische Großbuchstaben ohne "O" sowie Ziffern von 0 bis 9) bietet der SVC rund 42.000 Codevarianten, was in Kombination mit einer zeitlich begrenzten Zuordnung ausreicht, um sämtliche aktuell in Behandlung befindlichen Patienten oder sämtliche Bettplätze auch eines größeren Krankenhauses abzubilden. Somit kann bei der Patientenzuordnung von medizinischen Geräten auf einen Scanner oder auf ein anderweitiges Lesegerät (z. B. Smart Device) verzichtet werden.

Im Fall **CPA** gibt es folgende strukturelle Voraussetzungen / Randbedingungen und spezifische Vorteile:

### CPA-bedingte Neuerungen im Infusionspumpen-System

- Eine Eingabemöglichkeit für den Patienten-SVC an der Infusionspumpe
- Eine indirekt über das Backend des Infusionspumpensystems erfolgende Datenbankabfrage auf Basis des eingegebenen SVC
- Ein Anzeige- und Bestätigungsdialog für die auf Basis des Patienten-SVC ermittelte Patienten-ID

### CPA-bedingte Neuerungen im KIS oder EMR-System

- Eine Liste gültiger Patienten-SVCs, die jeweils temporär einer Patienten-ID oder einer Fallnummer zugeordnet werden (welche ihrerseits einer Patienten-ID zugeordnet ist).
- Eine Abfragemöglichkeit für die Patienten-ID oder Fallnummer sowie den Namen, das Alter und das Geschlecht des Patienten auf Basis des Patienten-SVC.
- Patientenarmbänder, auf denen neben der Patienten-ID zusätzlich der aktuell zugeordnete Patienten-SVC aufgedruckt ist.

### Vorteile des CPA-Systemkonzeptes:

- Kein Scanner, kein Smart Device und keine Interaktion mit weiteren Systemen ist für die Patientenzuordnung erforderlich. Dadurch ist eine Implementierung wesentlich einfacher und schneller umzusetzen. Auch der Wartungsaufwand und die Zahl der möglichen Fehlerquellen reduzieren sich.
- Die Zuordnung ist für einzeln genutzte Geräte ebenso möglich wie für Infusionspumpen, die in Racksystemen genutzt werden. Dabei spielt es keine Rolle, ob die Racksysteme ortsfest sind, oder nicht.
- Alle Fehlermöglichkeiten einer indirekten Zuordnung über den Bettplatz entfallen ersatzlos (Notfallpatient hat noch keinen Bettplatz, Zuordnung Patient <> Bettplatz erfolgt verzögert / ist nicht mehr aktuell... )
- Die Zuordnung erfolgt direkt an der Infusionspumpe, verglichen mit bestehenden Lösungen jedoch in einer äußerst einfachen und schnellen Interaktion (Eingabe eines bevorzugt dreistelligen Codes vom Patientenarmband, Bestätigung der zugeordneten Patientendaten).

Im Fall **CLA** gibt es folgende strukturelle Voraussetzungen / Randbedingungen und spezifische Vorteile:

### CLA-bedingte Neuerungen im Infusionspumpen-System

- Eine Eingabemöglichkeit für den Bettplatz-SVC an der Pumpe
- Im Fall A: Eine Datenbankabfrage der Pumpe an den für die Verwaltung der Hospital Structure zuständige Komponente des übergeordneten Infusionspumpen-Backends (Ausgabe der Bettplatz-Bezeichnung zum jeweiligen Location-SVC)
- Im Fall B: Eine indirekt über das Backend des Infusionspumpensystems erfolgende Datenbankabfrage an das für die Bettplatz-Zuordnung zuständige, übergeordnete EMR-System oder KIS (Ausgabe der Bettplatz-Bezeichnung zum jeweiligen Location-SVC)
- Im Fall A: Ein Anzeige- und Bestätigungsdialog für den auf Basis des Location-SVC ermittelten Bettplatz, inklusive Anzeige von bettplatzbezogenen Metadaten aus der Hospital Structure (z. B. Raum, Care Unit, Gebäude, etc.)
- Im Fall B: Ein Anzeige- und Bestätigungsdialog für den auf Basis des Location-SVC ermittelten Bettplatz

### CLA-bedingte Neuerungen im Infusionspumpen-Backend

- Im Fall A: Eine Liste gültiger und eindeutiger Bettplatz-SVCs, die jeweils einem Bettplatz ("Point of Care") in der Hospital Structure zugeordnet sind.
- Im Fall A: Eine System-interne Abfragemöglichkeit für die Bettplatz-Bezeichnung auf Basis des Location-SVC inkl. Rückgabe dazugehöriger Metadaten (z. B. Raum, Care Unit, Gebäude, etc.).
- Im Fall B: Eine Abfragemöglichkeit beim übergeordneten EMR-System oder KIS für die für die Bettplatz-Bezeichnung auf Basis des Location-SVC

### CLA-bedingte Neuerungen im KIS oder EMR-System

- Im Fall A: n/a
- Im Fall B: Eine Liste gültiger und eindeutiger Location-SVCs, die jeweils einem Bettplatz zugeordnet sind.
- Im Fall B: Eine Abfragemöglichkeit für die Bettplatz-Bezeichnung auf Basis des Bettplatz-SVC.

### CLA-bedingte Neuerungen im Krankenhaus bzw. Patientenzimmer

- Schilder, Aufkleber oder Ähnliches an jedem Bettplatz, an dem die Bettplatz-Bezeichnung sowie der dazugehörige Location- oder Bettplatz-SVC einfach abgelesen werden können.

### Vorteile des CLA-Systemkonzeptes:

- Die Zuordnung erfolgt direkt an der Infusionspumpe, verglichen mit bestehenden Lösungen jedoch in einer äußerst einfachen und schnellen Interaktion (Eingabe eines am Bettplatz abgelesenen zwei- oder dreistelligen Codes, Bestätigung der Zugeordneten Bettplatzes, ggfs. inkl. Metadaten).
- Kein Scanner, kein Smart Device und keine Interaktion mit weiteren Systemen erforderlich. Dadurch ist eine Implementierung wesentlich einfacher und schneller umzusetzen. Auch der Wartungsaufwand und die Zahl der möglichen Fehlerquellen reduzieren sich.
- Die Bettplatzzuordnung ist für einzeln genutzte Geräte ebenso möglich wie für Infusionspumpen, die in Racksystemen genutzt werden. Dabei spielt es keine Rolle, ob die Racksysteme ortsfest sind, oder nicht.
- Das Konzept deckt eine Bettplatzzuordnung innerhalb des Infusionspumpen-Backends (Fall A) ebenso ab, wie eine Zuordnung im übergeordneten EMR-System bzw. KIS (Fall B).

Im Ergebnis ermöglichen die erfindungsgemäße Vorrichtung und das Verfahren eine einfache und scannerlose Patienten- oder Bettplatzzuordnung von Infusionspumpen oder anderen medizinischen Geräten - als Ersatz sowohl für die indirekte (und in bestimmten Fällen kaum umsetzbare) traditionelle Zuordnung über den Bettplatz, als auch für die Listenauswahl am Gerät oder andere Workarounds.

Mehrere Ausführungsbeispiele der Erfindung werden nachfolgend anhand der beigefügten Zeichnung näher erläutert.

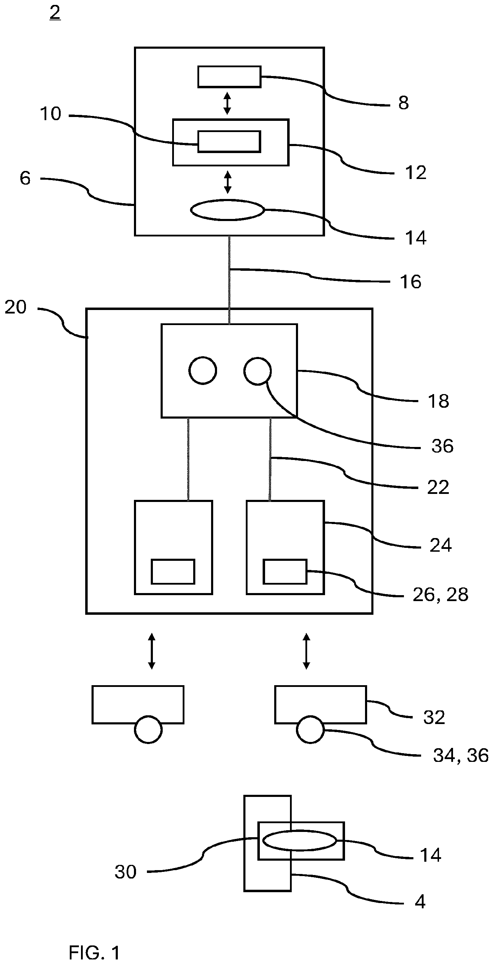

Darin zeigt FIG. 1 ein stark vereinfachtes Strukturdiagramm eines Behandlungszentrums zur Durchführung von medizinischen Infusionen.

Das in FIG. 1 in einem schematischen Überblick dargestellte medizinische Behandlungszentrum 2 ist zur Durchführung von medizinischen Infusionen an einer Vielzahl wechselnder Patienten 4 ausgelegt. Ein computergestütztes Verwaltungssystem 6, bei dem es sich beispielsweise um ein Krankenhaus-Informations-System (KIS) oder um ein auf elektronischen Gesundheitsakten (EMR = Electronic Medical Record) basiertes System handeln kann, beinhaltet eine Datenbank, in der Behandlungsfälle beispielsweise nach Fallnummern 8 sortiert abgespeichert sind. Jeder Fallnummer 8 ist ein Patient 4 zugeordnet, der durch eine eindeutige Patientenkennung oder Patienten-ID 10 identifizierbar ist. Dabei handelt es sich im Regelfall um eine längere Zeichenkette oder vielstellige Buchstaben- und/oder Ziffernkombination, konkret etwa: 08154711-1234. Die Patienten-ID 10 ist deshalb vergleichsweise lang, um auch über Jahre hinweg jeden Patienten 4, der das Behandlungszentrum 2 aufsucht, eindeutig kennzeichnen zu können. Jeder Patienten-ID 10 ist ein vollständiger Datensatz von Patientendaten 12 zugeordnet, der beispielsweise Namen, Alter und Geschlecht des Patienten umfasst.

Die Länge der Patienten-ID 10 macht eine fehlerfreie manuelle Eingabe in Daten verarbeitende Systeme oder Geräte schwierig. Andererseits reicht für eine vorgegebene Gültigkeitsdauer von beispielsweise vier Wochen eine weitaus kürzere Zeichen- und/oder Ziffernkette aus, um das typische Patientenaufkommen in dem Behandlungszentrum 2 auf eineindeutige Weise zu kennzeichnen. Aus diesem Grund generiert das Verwaltungssystem 6 für einen aktuell in Behandlung befindlichen oder dafür vorgesehenen Patienten 4 zu seiner Patienten-ID 10 gewissermaßen eine Kurzform, die ihn für eine vorgegebene Gültigkeitsdauer oder eine geplante Behandlungsdauer, beispielsweise einen Zeitraum von vier Wochen, neben anderen aktuell behandelten Patienten 4 eindeutig kennzeichnet. Dieser sogenannte Kurzkennzeichnungscode oder Kurzbestätigungscode (SVC = Short Verification Code), auch Patienten-SVC 14 genannt, ist bevorzugt ein aus wenigen Stellen bestehender Code, insbesondere ein alphanumerischer Code, bei dem jede der Stellen bevorzugt ausgewählt ist aus der Gesamtheit der lateinischen Großbuchstaben von A bis Z sowie den Ziffern 0 bis 9 oder aus einer Submenge davon (beispielsweise nur Buchstaben oder nur Ziffern). Wegen Verwechslungsgefahr wird vorteilhafterweise nicht zwischen "O" und "0" unterschieden. Besonders bevorzugt ist ein dreistelliger Code, insbesondere ein dreistelliger alphanumerischer Code, der von einem menschlichen Benutzer leicht zur erfassen und kurzzeitig zu merken ist und dennoch unter den genannten Voraussetzungen rund 42.000 Kombinationsmöglichkeiten zulässt. Um eine Mehrfachvergabe zu vermeiden, kann das Verwaltungssystem 6 die momentan vergebenen Patienten-SVCs 14 entsprechend markieren und/oder absondern, wobei nach Ablauf der Gültigkeitsdauer wieder eine Freigabe erfolgt.

Das Verwaltungssystem 6 übermittelt den Patienten-SVC 14, ggf. zusammen mit anderen Patientendaten 12, an einen nicht dargestellten Etikettendrucker oder an ein anderes Beschriftungssystem, so dass im Ergebnis ein vom Patienten 4 während des Aufenthaltes in der Behandlungseinrichtung 2 zu tragendes Patientenarmband 30 mit dem Patienten-SVC 14 und ggf. weiteren Informationen beschriftet wird.

Das Verwaltungssystem 6 ist über eine (kabelgebundene oder drahtlose) Datenverbindung 16 datenmäßig an ein Backend 18 eines Infusionspumpensystems 20 angeschlossen. An das Backend 18 wiederum ist über entsprechende (kabelgebundene oder drahtlose) Datenverbindungen 22 eine Mehrzahl von Infusionspumpen 24 anschließbar. Die räumliche Aufstellung der Infusionspumpen 24 im Behandlungszentrum 2 kann über die Zeit wechseln, und je nach Verfügbarkeit kann ein Patient 4 für eine Infusionsbehandlung, jedenfalls im Prinzip, einer beliebigen freien, an das Backend 18 angeschlossenen Infusionspumpe 24 zugewiesen werden.

Für einen sicheren, auf die individuellen Patientenbedürfnisse abgestimmten Betrieb mit entsprechender individueller Programmierung oder Dosierungseinstellung ist es jedoch notwendig, dass das Verwaltungssystem 6 und/oder das Backend 18 die Zuordnung zwischen Patient 4 und Infusionspumpe 24 kennt, und/oder dass die Infusionspumpe 24 den Patienten 4 "kennt" und beispielsweise ihm zugeordnete Dosierungsanweisungen oder dergleichen aus dem Verwaltungssystem 6 herunterladen kann. Zu diesem Zweck weist die jeweilige Infusionspumpe 24 eine Benutzeroberfläche mit einer Eingabemöglichkeit oder einem Eingabeelement 26 für den Patienten-SVC 14 des aktuell angeschlossenen Patienten 4 auf. Vorteilhafterweise handelt es sich dabei um eine graphische Benutzeroberfläche (GUI) mit einem Touchscreen 28, auf dem ein dediziertes Soft-Keyboard implementiert ist. Alternativ kann es sich aber auch um eine Hardware-Tastatur oder ein Hardware-Tastenfeld mit einem separaten Display handeln. Auch eine Eingabe über eine der Infusionspumpe 24 zugeordnete Tastatur an anderem Ort in der Nähe ist denkbar.

Ein Mitglied des behandelnden medizinischen Personals liest vom Patientenarmband 30 des an die Infusionspumpe 24 anzuschließenden oder bereits angeschlossenen Patienten 4 den Patienten-SVC 14 ab und gibt diesen über das Eingabeelement 26 ein. Die Infusionspumpe 24 sendet den Patienten-SVC 24 nun (über die Datenverbindungen 16, 22) über das Backend 18 des Infusionspumpen-Systems 20 an das Verwaltungssystem 6 und erhält im Gegenzug nach entsprechender Datenbankabfrage die aktuell damit verknüpfte Patienten-ID 10 sowie - optional - Patientendaten 12 wie den Namen, das Alter sowie das Geschlecht des Patienten 4.

Die letztgenannten Metadaten werden - neben der ermittelten Patienten-ID 10 - auf der Benutzerschnittstelle der Infusionspumpe 24 lediglich angezeigt, um dem Benutzer auf einfache Art ein zusätzliches Feedback zur Korrektheit der Zuordnung zu ermöglichen. Unmittelbar nach der positiven oder negativen Bestätigung des Benutzers werden diese Daten jedoch gelöscht; länger (in diesem Kontext heißt das zumindest für die vorgesehene Behandlungsdauer) in der Infusionspumpe 24 gespeichert wird lediglich die positiv verifizierte Patienten-ID 10, welche nach dem Start der Infusion dann auch in dem an das Verwaltungssystem 6 gesendeten Datenstrom enthalten ist.

Im Ergebnis ermöglicht die unkomplizierte Eingabe des vom Patientenarmband 30 abgelesenen Patienten-SVC 14 direkt an der Infusionspumpe 24 eine korrekte Zuordnung zwischen Patient 4 und Infusionspumpe 24 für die jeweilige Behandlung (Infusion). Dies ist möglich, ohne den genauen Aufstellungsort der Infusionspumpe 24 zu kennen. Im Vergleich zur dauerhaften Zuordnung zwischen Patienten-ID 10, Fallnummer 8 und Patientendaten 12 ist diese Zuordnung zwischen Patient 4 und Infusionspumpe 24 temporär und wird im Verwaltungssystem 6 entsprechend häufig bei Änderung aktualisiert - wie auch der allenfalls ein paar Wochen gültige Patienten-SVC 14.

In einer Abwandlung des beschriebenen Konzepts werden die Infusionspumpen 24 an dedizierten Behandlungsorten, insbesondere Bettplätzen 32, aufgestellt. Der Einfachheit halber wird im Folgenden von Bettplätzen 32 gesprochen, wobei der allgemeinere Fall eines feststehenden (unbeweglichen) Behandlungsortes aber immer mit enthalten ist. Der jeweilige Bettplatz 32, an den eine zugehörige Infusionspumpe 24 angeschlossen ist, ist mit einer Beschriftung 34 oder Kennzeichnung versehen, die eine innerhalb des Behandlungszentrums 2 eindeutige Bettplatzbezeichnung oder Bettplatzbeschreibung im Klartext und einen eineindeutig zugeordneten Bettplatz-SVC 36 (allgemeiner: Behandlungsort-SVC oder Location-SVC) als Kurzfassung davon enthält.

Für den Bettplatz-SVC 36 gilt das oben für den Patienten-SVC 14 Gesagte, so dass beispielsweise ein dreistelliger alphanumerischer Code aus lateinischen Großbuchstaben von A bis Z sowie Ziffern 0 bis 9 (wegen Verwechslungsgefahr werden zweckmäßigerweise "O" und "0" synonym verwendet) rund 42.000 Möglichkeiten für eine eindeutige Bettplatzbezeichnung generiert - mehr als ausreichend viele selbst für die allergrößten Krankenhäuser.

Es wird unterstellt, dass die planmäßige, grundsätzliche Zuordnung zwischen Bettplatz 32 und Infusionspumpe 24 im Backend 18 des Infusionspumpensystems 20 bekannt ist, dort also abgespeichert ist, vorzugsweise ebenso die Bettplatzbeschreibung.

Wurde ein Patient 4 durch ein Mitglied des medizinischen Personals oder einen sonstigen Nutzer einem freien Bettplatz 32 zugewiesen, so liest der Nutzer aus der zugehörigen Beschriftung 34 den Bettplatz-SVC 36 ab und gibt diesen über ein Eingabeelement 26 an der Infusionspumpe 24 ein. Wie schon im Zusammenhang mit dem Patienten-SVC 14 beschrieben, kann es sich dabei bevorzugt um ein Soft-Keyboard als Bestandteil einer graphischen Benutzeroberfläche oder alternativ um ein Hardwaretastenfeld handeln.

Der an der Infusionspumpe 24 eingegebene Bettplatz-SVC 36 wird über die Datenverbindung 22 an das Backend 18 übermittelt. Im Backend 18 wird gemäß Raumausstattungsplan oder Strukturplan (Hospital Structure) des Behandlungszentrums 2 die zugehörige Bettplatzbezeichnung mittels Datenbankabfrage herausgesucht und im Erfolgsfall auf dem Display der Infusionspumpe 24 zur Bestätigung angezeigt (vorzugsweise inklusive Metadaten). Nach erfolgter Bestätigung am Eingabeelement 26 der Infusionspumpe 24 wird im Backend 18 die konkrete Zuordnung zwischen Infusionspumpe 24 und Bettplatz 32 verbindlich vorgenommen und die Bettplatzbezeichnung zusammen mit den Infusionsdaten an die nachgelagerten Systeme übermittelt.

In einer alternativen Variante davon ist die planmäßige Zuordnung zwischen Bettplatz 32 und Infusionspumpe 24 im Verwaltungssystem 6 gespeichert, und das Backend 18 fragt die Information dort ab. Im Erfolgsfall wird diese auf dem Display der Infusionspumpe 24 zur Bestätigung angezeigt. Auch hier wird nach erfolgter Bestätigung am Eingabeelement 26 der Infusionspumpe 24 die empfangene Bettplatzbezeichnung zusammen mit den Infusionsdaten an die nachgelagerten Systeme übermittelt.

Denkbar ist es auch, dass an der Infusionspumpe 24 lediglich der Bettplatz-SVC 36 eingegeben und direkt ohne Bestätigung verwendet wird, das heißt zusammen mit den Infusionsdaten an die nachgelagerten Systeme übermittelt wird. Der User erhält dann allerdings keine Rückmeldung, auf deren Basis er die Korrektheit seiner Eingabe überprüfen könnte. Insofern ist diese Art der Bettplatzzuordnung nicht so sicher wie die zuvor beschriebenen Varianten.

Prinzipiell ist es möglich, die auf der Patienten-SCV 14 und auf der Bettplatz-SVC 36 beruhenden Strukturen und Verfahren für sich genommen zu implementieren oder alternativ miteinander zu kombinieren.

Anstelle eines Armbands kann auch ein Halsband oder ein Fußband oder ein anderes körpergetragenes, zweckmäßigerweise nicht ohne Zerstörung entfernbares Identifikationsband oder Identifikationsmerkmal, etwa auch ein Kleidungstück, zum Einsatz kommen.

Die beschriebenen Konzepte lassen sich nicht nur für Infusionspumpen anwenden, sondern im Prinzip für beliebige Medizingeräte, die patientenspezifische Einstellungen erlauben.

### Bezugszeichenliste

- 2: Behandlungszentrum
- 4: Patient
- 6: Verwaltungssystem
- 8: Fallnummer
- 10: Patienten-ID
- 12: Patientendaten
- 14: Patienten-SVC
- 16: Datenverbindung
- 18: Backend
- 20: Infusionspumpensystem
- 22: Datenverbindung
- 24: Infusionspumpe
- 26: Eingabeelement
- 28: Touchscreen
- 30: Patientenarmband
- 32: Bettplatz
- 34: Beschriftung
- 36: Bettplatz-SVC

## Patentansprüche

1. Behandlungszentrum (2) zur Durchführung von medizinischen Infusionen, umfassend:
a. ein Verwaltungssystem (6) mit einer Datenbank zur Speicherung von Patientendaten (12), wobei jedem Patienten (4) eine Patienten-ID (10) oder eine Fallnummer (8) zugeordnet ist,
b. ein Infusionspumpensystem (20) mit einem Backend (18), das über Datenverbindungen (16, 22) mit dem Verwaltungssystem (6) und mit einer Mehrzahl von Infusionspumpen (24) verbunden ist,
c. eine Mehrzahl von Infusionspumpen (24), jeweils mit einer Benutzeroberfläche einschließlich eines Eingabeelements (26), das die Eingabe eines Kurzkennzeichnungscodes (14), SVC genannt, ermöglicht.
d. eine Mehrzahl von körpergetragenen Identifikationsmerkmalen, insbesondere Patientenarmbändern (30), jeweils mit einem Patienten-SVC (14) beschriftet, zur temporären, eindeutigen Kennzeichnung von Patienten (4) für einen vorgegebenen Behandlungszeitraum, oder
eine Mehrzahl von jeweils einer der Infusionspumpen (24) zugeordneten Bettplätzen (32), jeweils mit einem eindeutigen Bettplatz-SVC (36) beschriftet,
wobei das Verwaltungssystem (6) und/oder das Backend (18) dazu ausgebildet ist, anhand eines an einer der Infusionspumpen (24) eingegebenen Patienten-SVCs (14) oder Bettplatz-SVCs (36) eine Zuordnung zwischen Patient (4) und Infusionspumpe (24) oder zwischen Bettplatz (32) und Infusionspumpe (24) vorzunehmen.

2. Behandlungszentrum (2) nach Anspruch 1, wobei der jeweilige SVC ein zwei- oder dreistelliger alphanumerischer Code ist.

3. Behandlungszentrum (2) nach einem der vorangehenden Ansprüche, wobei das Verwaltungssystem (6) dazu ausgebildet, den Patienten-SVC (14) zu generieren und diesen zusammen mit anderen Patientendaten (12) an einen Etikettendrucker oder ein Beschriftungssystem zu übermitteln, um das körpergetragene Identifikationsmerkmal zu beschriften.

4. Behandlungszentrum (2) nach einem der vorangehenden Ansprüche, wobei das Verwaltungssystem (6) eine Liste gültiger Patienten-SVCs (14), die jeweils temporär einer Patienten-ID (10) oder einer Fallnummer (8) zugeordnet werden, enthält.

5. Behandlungszentrum (2) nach einem der vorangehenden Ansprüche wobei das Verwaltungssystem (6) eine Liste gültiger Bettplatz-SVCs (36), die jeweils einem Bettplatz (32) des Behandlungszentrums (2) zugeordnet werden, enthält.

6. Behandlungszentrum (2) nach einem der vorangehenden Ansprüche, wobei das Backend (18) die Zuordnung zwischen Bettplätzen (32) und Infusionspumpen (24) gemäß den Bettplatz-SVCs (36) verwaltet und aktualisiert.

7. Behandlungszentrum (2) nach einem der vorangehenden Ansprüche, wobei das Verwaltungssystem (6) die Zuordnung zwischen Patienten (4) und Infusionspumpen (24) gemäß den Patienten-SVCs (14) oder die Zuordnung zwischen Bettplätzen (32) und Infusionspumpen (24) gemäß den Bettplatz-SVCs (36) verwaltet und aktualisiert.

8. Behandlungszentrum (2) nach einem der vorangehenden Ansprüche, wobei die jeweilige Infusionspumpe (24) eine graphische Benutzeroberfläche mit einem Touchscreen (28) aufweist, der das Eingabeelement (26) für den SVC bildet.

9. Behandlungszentrum (2) nach einem der vorangehenden Ansprüche, wobei das Verwaltungssystem (6) dazu ausgebildet ist, nach Erhalt eines SVCs einen Satz von Patientendaten (12) oder eine Bettplatzbeschreibung zwecks Anzeige und Bestätigung durch einen Benutzer an die betreffende Infusionspumpe (24) zu senden.

10. Verfahren zum Betreiben eines Infusionspumpensystems (20) in einem Behandlungszentrum (2) nach einem der vorangehenden Ansprüche, bei dem das Verwaltungssystem (6) und/oder das Backend (18) anhand eines an einer der Infusionspumpen (24) eingegebenen Patienten-SVCs (14) oder Bettplatz-SVCs (36) eine Zuordnung zwischen Patient (4) und Infusionspumpe (24) oder zwischen Bettplatz (32) und Infusionspumpe (24) vornimmt.
